# EUROPEAN PATENT SPECIFICATION

(11) **EP 2 554 656 B1**
(45) Date of publication and mention of the grant of the patent: **16.05.2018**
(21) Application number: 11759611.4
(22) Date of filing: 25.03.2011
(51) Int. Cl.: C12N 1/16, C12P 21/02, A23L 27/24, A23L 33/14, A23L 33/145

(54) **METHOD FOR CULTURING YEAST**
VERFAHREN ZUR KULTIVIERUNG VON HEFE
PROCÉDÉ DE CULTURE DE LEVURE

(30) Priority: 26.03.2010 JP 2010073818
(43) Date of publication of application: 06.02.2013
(73) Proprietor: Asahi Group Holdings, Ltd., Tokyo 130-8602 (JP)
(72) Inventor: TANAKA Miwa, Moriya-shi Ibaraki 302-0106 (JP); KAWASHIMA Kenji, Moriya-shi Ibaraki 302-0106 (JP)
(74) Representative: Desaix, Anne
(86) International application number: PCT/JP2011/057446
(87) International publication number: WO 2011/118807

(56) References cited:
- WO-A1-95/23857
- WO-A1-2006/013736
- JP-A- 48 019 793
- JP-A- 2000 279 164
- JP-A- 2006 042 638
- JP-A- 2009 107 962
- IKENO Y ET AL: "Glutathione prepd. by culturing a Candida or Pichia yeast - in a medium contg. L-cystine", WPI / THOMSON,, vol. 1977, no. 3, 1 December 1976 (1976-12-01), XP002607290,
- ADAMA, C. ET AL.: 'Optimization of biomass and dihydroorotase (DHOase) production by Saccharomyces cerevisiae MNJ3 (pMNJ1)' AFR.J. BIOTECHNOL. vol. 8, no. 1, 2009, pages 37 - 41, XP008161440
- NIELSEN, M.K. ET AL.: 'The effect of citric acid and pH on growth and metabolism of anaerobic Saccharomyces cerevisiae and Zygosaccharomyces bailii cultures' FOOD MICROBIOL. vol. 24, no. 1, 2007, pages 101 - 105, XP005593084
- LIANG, G. ET AL.: 'Elevated glutathione production by adding precursor amino acids coupled with ATP in high cell density cultivation of Candida utilis' J.APPL. MICROBIOL. vol. 105, no. 5, 2008, pages 1432 - 1440, XP008161439

## Description

### TECHNICAL FIELD

The present disclosure relates to: a method of culturing a yeast, whereby the glutathione content in the yeast cells can be increased; and a method of producing a yeast extract from a yeast cultured by the aforesaid method. The invention more particularly relates to a method of increasing a glutathione content of a yeast, as claimed in claim 1.

Priority is claimed on Japanese Patent Application No. 2010-073818 filed March 26,2010.

### BACKGROUND ART

Yeast belonging to the genus *Saccharomyces* typified by brewing yeast and baker's yeast, contains a good balance of natural B vitamins, amino acids, minerals, and so forth. This yeast is usefully applied not only to the bakery and brewery productions but also to many other utilities. For example, in Japan, dry yeast has been used as a drug, a food material, a seasoning, and such applications, over a long period of time. This yeast has been appreciated as highly nutritious and safe materials. In addition, in recent years, yeast is widely used as a starting material of yeast extract.

Yeast extract means a preparation made from a culture product of a yeast. It abundantly contains amino acids and other compounds. Yeast extracts have been so far used as food additives such as seasonings for enriching the taste and the flavor. Particularly, because of today's growing appreciation for natural products, the demand for yeast extracts as seasonings tends to increase. It is expected that yeast extracts prepared from such yeast which abundantly contain taste components, can be used as much better seasonings. Therefore, the development of yeast that contains greater amounts of taste components has been actively carried out.

As representative examples of sulfur-containing compounds contained in yeast cells, glutathione and S-adenosylmethionine can be enumerated. Glutathione is an extremely useful substance that has an effect of recovering the hepatic function, and has an antioxidative activity, and the like. In recent years, it is expected to use glutathione for a wide range of applications including the use as seasonings and additives for foods and drinks including health foods, substrate materials for cosmetics, and so forth. On the other hand, S-adenosylmethionine is known to have a function as a methyl group donor in various kinds of biological reactions. What is more, other effects such as an anti-depressant effect, an effect of alleviating arthrosis, and an effect of recovering the hepatic function have been reported. These sulfur-containing compounds are known to play important roles for living bodies.

Sulfur-containing compounds are usually synthesized from transcription and translation products of many genes typified by the MET gene (methionine biosynthesis gene) group, with use of sulfur-containing amino acids such as methionine and cysteine. Therefore, in order to obtain highly productive yeast for sulfur-containing compounds, it has been widely conducted to mutate genes of yeast related to the synthesis of these sulfur-containing compounds, so as to produce mutant strains of yeast which have high contents of sulfur-containing compounds. For example, as a method of producing yeast of high glutathione content, disclosed is a method (1) which includes conducting a mutagenic treatment and aerobically culturing mutant strains of yeast belonging to the genus *Candida* that have been made viable in a medium containing both ethionine and sulfite, so as to thereby increase the glutathione content in the yeast cells (for example, refer to Patent Document 1).

### [Prior Art Document]

### [Patent Document]

Patent Document 1: Japanese Unexamined Patent Application, First Publication No. Sho 59-151894.

It is also known from Ikeno Y et al "Gutathione prepared by culturing a Candida or Pichia yeast in a medium containing L-cystine" WPI/THOMPSON, vol 1977, no. 3, 1 December 1976, a method for recovering yeast comprising an increased amounts of glutathione, the level of which is enhanced because of the presence of L-cystine.

### DISCLOSURE OF INVENTION

### PROBLEMS TO BE SOLVED BY THE INVENTION

In order to conduct industrial mass production of glutathione itself, or yeast extracts which abundantly contain glutathione, and the like, in a more efficient and less expensive way, it is important to make use of yeast having high glutathione contents.

Although it is possible to obtain yeast of high glutathione content by the method as described in Patent Document 1 which includes conducting a mutagenic treatment or such a treatment followed by screening for yeasts having higher glutathione contents among the thus genetically modified yeast, it takes time and effort to conduct such mutagenic treatment and screening, and what is worse, yeasts of high glutathione content are not always obtainable in many cases.

In addition, natural yeast (wild-type strain) is sometimes more demanded than recombinants. It is thus desired to make a development of the method of increasing the glutathione content in a yeast without conducting a mutagenic treatment. For example, when a yeast itself or a yeast extract is used as a food, the wild-type strain is often more preferred than recombinants.

It is an object of the present disclosure or invention to provide a method of culturing a yeast or a method of increasing a glutathione content of a yeast, whereby the glutathione content in the yeast cells can be increased without applying genetic modifications.

### MEANS TO SOLVE THE PROBLEMS

The inventors of the present invention have conducted intensive studies to solve the above-mentioned problems. As a result, they discovered that the glutathione content of a yeast such as a yeast belonging to the genus *Saccharomyces* can be increased by adding a predetermined amount or more of citric acid to a liquid medium for culturing the yeast. This has led to the completion of the present invention.

That is to say, the present disclosure provides:
(1) a method of culturing a yeast including culturing a yeast in a liquid medium whose citric acid concentration is equal to or greater than 20 mM;
(2) the method of culturing a yeast according to (1), wherein the citric acid concentration of the liquid medium is equal to or lower than 200 mM;
(3) the method of culturing a yeast according to (1) or (2), wherein the citric acid concentration of the liquid medium at the initiation of the culture is equal to or greater than 20 mM;
(4) the method of culturing a yeast according to (1), wherein the citric acid concentration of the liquid medium at the initiation of the culture is lower than 20 mM, and the method includes adjusting the citric acid concentration of the liquid medium at 20 to 200 mM when the proliferative status of the yeast is in an induction phase or a logarithmic growth phase;
(5) the method of culturing a yeast according to (1), wherein the citric acid concentration of the liquid medium at the initiation of the culture is lower than 20 mM, and the method includes adjusting the citric acid concentration of the liquid medium at 20 to 200 mM within 9 hours after the initiation of the culture;
(6) the method of culturing a yeast according to any one of (1) to (5), wherein the yeast is a yeast belonging to the genus *Saccharomyces* or a yeast belonging to the genus *Candida*;
(7) the method of culturing a yeast according to any one of (1) to (5), wherein the yeast is *Saccharomyces cerevisiae* or *Candida utilis*;
(8) a method of increasing a glutathione content of a yeast including culturing a yeast in a liquid medium whose citric acid concentration is equal to or greater than 20 mM;
(9) the method of producing a yeast including recovering a yeast cultured by the method of culturing a yeast according to any one of (1) to (7);
(10) a method of producing a yeast extract including extracting a yeast extract from a yeast cultured by the method of culturing a yeast according to any one of (1) to (7);
(11) a method of producing a food or drink including using, as a starting material, one or more products selected from the group consisting of a yeast cultured by the method of culturing a yeast according to any one of (1) to (7) and a yeast extract prepared from the yeast;
(12) a yeast extract prepared from a yeast cultured by the method of culturing a yeast according to any one of (1) to (7);
(13) a seasoning composition including a yeast cultured by the method of culturing a yeast according to any one of (1) to (7), or the yeast extract according to (12); and
(14) a food or drink including a yeast cultured by the method of culturing a yeast according to any one of (1) to (7), or the yeast extract according to (12).

The invention more particularly relates to a method of increasing a glutathione content of a yeast, as defined in claim 1.

### EFFECTS OF THE INVENTION

The method of culturing a yeast of the present disclosure is capable of increasing the glutathione content of a yeast belonging to the genus *Saccharomyces* or such a yeast by a simple step of culturing it in a liquid medium containing a sufficient amount of citric acid. In addition, the yeast cultured by this culturing method has sufficiently high glutathione content. Thus, a yeast extract or a food or drink having a high glutathione content can be readily and conveniently obtained by using this yeast.

### BRIEF DESCRIPTION OF THE DRAWINGS

FIG. 1 is a graph showing the GSH content rate (%) with respect to the concentration of citric acid in a liquid medium in Example 1.
FIG. 2A is a graph showing the GSH content rate (%) of respective culture products with respect to the concentration of citric acid added to a molasses medium (1) in Example 2.
FIG. 2B is a graph showing the pH of the liquid medium at the completion of the culture (final pH) with respect to the concentration of citric acid added to the molasses medium (1) in Example 2.
FIG. 3A shows the measurement results of the GSH content rate and OD₆₀₀ of *Saccharomyces cerevisiae* strain KK122 in Example 5.
FIG. 3B shows the measurement results of the GSH content rate and OD₆₀₀ of *Saccharomyces cerevisiae* strain KK124 in Example 5.
FIG. 4 shows the measurement results of the GSH content rate (%) of yeasts in respective samples in Example 11.
FIG. 5 shows the measurement results of the citric acid content in respective supernatants before and after the culture in Reference Example 1.

### PREFERRED MODES FOR CARRYING OUT THE METHODS DISCLOSED

### HEREIN

In the present invention and the description of this application, unless otherwise stated, the term glutathione means both oxidized glutathione and reduced glutathione, and the term total glutathione content means the total content of oxidized glutathione and reduced glutathione.

In the present invention and the description of this application, the glutathione content per dry cell weight of a yeast can be obtained by a usual method of quantifying the glutathione content in a microorganism. For example, the total glutathione content per dry cell weight of a yeast can be measured according to the method of Titze et. al. (Analytical Biochemistry, Vol. 27, p. 502, 1969). This method is to measure the glutathione amount based on the fact that, in a reduction reaction in which 5,5'-dithiobis (2-nitrobenzoic acid) (DTNB) is reduced by nicotinamide adenine dinucleotide phosphate (reduced form; NADPH), the reaction rate of the reaction is proportional to the amount of existing glutathione.

The method of culturing a yeast disclosed herein is characterized in culturing the yeast in a liquid medium whose citric acid concentration is equal to or greater than 20 mM. It becomes possible, by culturing a yeast using this culturing method, to increase the glutathione content of the yeast and to obtain a high glutathione content yeast (meaning a yeast which has high glutathione content). The reason why such a glutathione-increasing effect (meaning an effect of increasing the glutathione content of a yeast) can be achieved is not clear. However, as shown in Example 3 that will be described later, considering the fact that this effect was not observed in cases of the addition of other types of acids to liquid media, and the fact that this effect was achieved by the addition of citric acid even under pH-controlled conditions, it is suggested that this effect was not merely due to the pH adjusting effect of the liquid medium, but rather due to the accumulation of glutathione in the yeast cells, which might be attributed to some kind of action peculiar to citric acid that works to promote the glutathione production or to keep glutathione from being discharged to the outside the cells.

One aspect of the method of culturing a yeast disclosed herein is a method of producing a yeast of high glutathione content, wherein the method includes culturing the yeast in a liquid medium whose citric acid concentration is equal to or greater than 20 mM so as to thereby obtain a culture product containing the yeast, and recovering the yeast from the culture product.

The term yeast of high glutathione content of the present disclosure refers to a yeast whose glutathione content in the yeast cells is significantly increased higher than that of the parent strain.

In addition, the term culture product means a culture product which contains the cell bodies and the medium used for the culture of the yeast.

The yeast to be supplied to the method of culturing a yeast of the present disclosure is not specifically limited, although preferred is a yeast belonging to the genus *Saccharomyces* or a yeast belonging to the genus *Candida.* For example, *Saccharomyces cerevisiae, Saccharomyces paradoxus, Saccharomyces mikatae, Saccharomyces bayanus, Saccharomyces kudriavzevii, Candida utilis, Candida tropicalis, Candida lypolitica, Candida sake,* or the like, can be employed. It is preferable to apply to the culture of *Saccharomyces cerevisiae* or *Candida utilis* because they can exhibit a particularly excellent glutathione-increasing effect. In the present invention, the yeast belongs to *Saccharomyces cerevisiae.*

The method of culturing a yeast of the present disclosure or invention is able to achieve the glutathione-increasing effect not only in cases of culturing wild-type strains (natural yeasts), but also in cases of culturing mutant strains obtained from a mutagenic treatment. Note that, the term "wild-type strain" in the description of this application means a yeast which has been originally present in nature, in other words, a yeast to the gene(s) of which has(have) not been applied any artificial mutagenic treatment. On the other hand, the term "mutant strain" means a yeast obtained by applying an artificial mutagenic treatment to the gene(s).

The mutagenic treatment in the present disclosure or invention is not specifically limited as long as it is a treatment that can cause a mutation in a part of gene(s) of a yeast or such an organism. Any usual technique for use in the production of mutant strains of a yeast or such a microorganism may be employed. For example, a mutagenic treatment onto a yeast can be carried out by treating the yeast with, as a mutagen, ultraviolet radiation, ionizing radiation, a nitrite, nitrosoguanidine, ethyl methanesulfonate (hereunder, abbreviated as EMS), or the like.

The citric acid concentration of the liquid medium for use in the method of culturing a yeast of the present disclosure may be any concentration as long as it is equal to or greater than 20 mM. By having the citric acid concentration equal to or greater than 20 mM, a sufficient amount of citric acid can be applied to the yeast enough to exert the glutathione-increasing effect. In the present disclosure or invention, the citric acid concentration of the liquid medium is preferably from 20 to 200 mM, more preferably from 20 to 120 mM, yet more preferably from 20 to 100 mM, and particularly preferably from 50 to 100 mM. If an excessive amount of citric acid is added to the liquid medium, the achieved level of the glutathione-increasing effect may not be as high as expected, and what is worse, there might be a risk of inhibiting the viability of the yeast or such an adverse event. However, if the citric acid concentration of the liquid medium is set equal to or lower 200 mM, a sufficient level of the glutathione-increasing effect can be achieved while suppressing the influence on the viability of the yeast. The invention is more particularly defined using the citric acid concentration values set forth in claim 1.

When the culture is conducted under a condition where the pH of the liquid medium is not controlled, even if another type of acid differing from citric acid has been added, the glutathione content of the liquid medium gets slightly higher than that of the liquid medium without the addition of any acid. The reason can be suggested that the pH of the liquid medium has been adjusted by the buffering action induced by the addition of the acid. That is to say, when the pH is not controlled, a part of the citric acid added to the liquid medium might be used for controlling the pH of the liquid medium. For this reason, the citric acid concentration of the liquid medium required for achieving the same level of the glutathione-increasing effect tends to be higher in pH-uncontrolled culture conditions rather than in pH-controlled culture conditions.

Therefore, in a case where the pH of the liquid medium is not controlled, it is preferable in the method of culturing a yeast of the present disclosure or invention to culture the yeast in a liquid medium having a citric acid concentration from 60 to 110 mM, and more preferably from 75 to 90 mM. On the other hand, in a case where the pH of the liquid medium is controlled to be from 4.0 to 6.0, it is preferable in the method of culturing a yeast of the present disclosure or invention to culture the yeast in a liquid medium having a citric acid concentration from 20 to 100 mM, and more preferably from 20 to 75 mM.

In the method of culturing a yeast of the present disclosure, it is either possible to culture the yeast with use of a liquid medium that has been previously prepared so that the citric acid concentration would be equal to or greater than 20 mM, or to add citric acid to a liquid medium after the initiation of the culture. In other words, it is either possible that: the yeast is cultured in a liquid medium whose citric acid concentration at the initiation of the culture is set equal to or greater than 20 mM; or that the yeast is cultured in a liquid medium whose citric acid concentration at the initiation of the culture is lower than 20 mM (including a liquid medium which contains no citric acid at all), and then, after the initiation of the culture, the citric acid concentration of the liquid medium is adjusted at 20 to 200 mM.

Regarding the method of adjusting the citric acid concentration of the liquid medium, it is either possible to make the adjustment by adding a solid citric acid to the liquid medium, or by adding an aqueous solution of citric acid thereto. The invention is more particularly defined through the adjustment step set forth in claim 1.

It tends to be more possible for yeasts belonging to the genus *Saccharomyces* or the genus *Candida,* and in particular, many strains belonging to the genus Saccharomyces, to achieve higher glutathione-increasing effect, as the timing of adding citric acid to the liquid medium is earlier. The glutathione-increasing effect can be sufficiently exerted by applying sufficient concentration of citric acid to an actively budding yeast. For this reason, if the citric acid concentration of the liquid medium is adjusted after the initiation of the culture, it is preferable to make an adjustment before the transition to the stationary phase, in other words, during a time when the proliferative status of the yeast is in the induction phase or the logarithmic growth phase, preferably during a time after the initiation of the culture until the middle stage of the logarithmic growth phase, and more preferably, during a time after the initiation of the culture until the early stage of the logarithmic growth phase.

Note that the term logarithmic growth phase refers to a phase in which, when it comes to batch and feeding culture, a logarithmic increase of the amount of a yeast in a culture vessel is observed if measured in a time course manner with reference to the absorbance as an index.

On the other hand, this term refers to a phase in which, when it comes to continuous culture, a substantially constant amount of a yeast in a culture vessel is observed.

The term induction phase refers to a phase after the initiation of the culture until reaching the logarithmic growth phase, when it comes to batch and feeding culture.

On the other hand, this term refers to a phase until controlling a parameter serving as a reference index to a constant value, when it comes to continuous culture.

For example, it is possible to apply a sufficient concentration of citric acid to an actively budding yeast by making an adjustment of the citric acid concentration of the liquid medium within 9 hours, preferably 3 hours, after the initiation of the culture.

The liquid medium for use in the method of culturing a yeast of the present disclosure or invention can be prepared by appropriately adding citric acid to a liquid medium, where a yeast can grow, so that the citric acid concentration would be equal to or greater than 20 mM.

The liquid medium to be added with the citric acid may be any type of medium which contains a carbon source, a nitrogen source, an inorganic salt, and the like, and which is for use for a usual culture of *Saccharomyces cerevisiae* or such a yeast.

The carbon source to be contained in the liquid medium can be exemplified by one or more materials selected from the group consisting of glucose, sucrose, acetic acid, ethanol, molasses, spent sulfite liquor, and the like, which are to be used for a usual culture of a microorganism. In addition, the nitrogen source may be either a nitrogen-containing inorganic salt or a nitrogen-containing organic material. For example, one or more materials selected from the group consisting of urea, ammonia, ammonium sulfate, ammonium chloride, ammonium phosphate, corn steep liquor (CSL), casein, yeast extract, peptone, and the like, can be adopted. Moreover, the inorganic salt can be exemplified by a phosphate component such as calcium superphosphate or ammonium phosphate, a potassium component such as potassium chloride or potassium hydroxide, a magnesium component such as magnesium sulfate or magnesium chloride, or the like. In addition, an inorganic salt of zinc, copper, manganese, ferrous ion, or the like, may also be adopted. Furthermore, to the liquid medium for use in the method of culturing a yeast of the present disclosure or invention may also be appropriately added a vitamin and a nucleic acid-related substance, and the like.

The liquid medium for use in the method of culturing a yeast of the present disclosure or invention may be a synthetic medium such as an SD medium, or a semisynthetic medium such as a YPD medium and a molasses medium. Alternatively, it may also be a medium like a molasses medium which naturally includes a small amount of citric acid. Furthermore, media prepared by modifying the above-mentioned media may also be adopted. A molasses medium (sugar concentration of 3%) generally has a total organic acid concentration of about 1 to 3.5 g/L and a citric acid concentration of 50 to 400 mg/L, although the content may differ depending on the lot, or the like. That is to say, it can be calculated from the molecular weight 192 of citric acid that the molasses medium to be used for the culture of a yeast usually contains 0.2 to 2 mM citric acid.

It has heretofore been known that citric acid can be used as a carbon source for culturing a yeast. However, in general, intentional addition of citric acid to a medium (for example, addition of citric acid to be equal to or greater than 10 mM) has not been done because citric acid has an influence on the pH of the medium. Furthermore, as shown in Reference Example 1 that will be described later, the amount of citric acid in the liquid medium after the culture increases to higher than that in the medium before the culture. Therefore, it is apparent that citric acid having been added to the liquid medium is not utilized as a carbon source in the present invention. Furthermore, it is the first finding discovered by the inventors of the present invention that the addition of citric acid to a liquid medium leads to an increase of the glutathione content in a yeast.

The manner of culture is not specifically limited as long as a liquid medium is used. The manner can be appropriately determined with consideration of the culture scale, the purpose of the application of the resulting culture product, and the like. The manner of culture in a liquid medium can be exemplified by batch culture, feeding culture, continuous culture, and the like.

The culture condition of the method of culturing a yeast of the present disclosure or invention is not specifically limited except for using a liquid medium which contains a predetermined concentration of citric acid. The culture can be conducted under a condition to be applied to general culture of a yeast. For example, the temperature of the culture is preferably from 20 to 40°C, and more preferably from 25 to 35°C. The pH of the medium is preferably from 3.5 to 8.0, and more preferably from 4.0 to 6.0. In particular, in a case of industrial mass production of a culture product, it is preferable to periodically check and adjust the pH in the medium so as to keep the pH at 4.0 to 6.0.

In addition, it is preferable to conduct the culture with aeration and shaking. The amount of aeration and the condition of shaking can be appropriately determined with consideration of the volume, the duration of the culture, and the initial concentration of the yeast. For example, the aeration can be carried out at about 0.2 to 2 V.V.M. (volume per volume per minute) and the shaking can be carried out at about 50 to 800 rpm.

The method of culturing a yeast of the present disclosure or invention is capable of culturing a yeast having a high glutathione content. For example, it is possible, by conducting culture by using the method of culturing a yeast of the present disclosure or invention, to increase the glutathione content in the dry yeast cells by 10% or higher than the case of conducting the culture in a liquid medium whose citric acid concentration is lower than 20 mM (including a liquid medium which contains no citric acid at all).

The term dry cell weight means the weight of dried cell bodies. The method of obtaining the weight of dried cell bodies can be exemplified by a method such that: for example, firstly, the culture product of a yeast is centrifuged so as to recover the cell bodies in the form of precipitation. The thus recovered cell bodies are washed with water by centrifugal manipulation two times, and then dried at 105°C for 5 hours. The dried cell bodies are then weighed. By so doing, the dry cell weight can be obtained.

That is to say, a culture product containing a yeast of a high glutathione content can be obtained by the method of culturing a yeast disclosed herein which includes culturing the yeast in a liquid medium whose citric acid concentration is equal to or greater than 20 mM, and the yeast can be recovered from the culture product by a known method.

The method of recovering the yeast from the culture product is not specifically limited, and any recovery method which is usually conducted for recovering a yeast from a culture product of the yeast can be adopted. The method of recovering the yeast from the culture product can be exemplified by a method of centrifuging the culture product of the yeast, or the like.

A yeast having a very high glutathione content can be produced by recovering the yeast from the culture product obtained by the method of culturing a yeast disclosed herein. In addition, the yeast extract can be prepared by extracting a yeast extract from the yeast cultured by the method of culturing disclosed herein. Accordingly, a yeast extract having a high glutathione content can be prepared and produced from the yeast produced by the method of culturing a yeast disclosed herein. As described above, glutathione is a substance having various physiological activities. In other words, a yeast and a yeast extract with higher added value than ever before can be produced simply by applying the method of culturing a yeast disclosed herein instead of a conventional culturing method.

The preparation of a yeast extract from the yeast cultured by the method of culturing a yeast disclosed herein is not specifically limited, and any preparation method which is usually conducted for preparing a yeast extract can be adopted. The preparation method can be exemplified by the autolysis method of lysing cell bodies with the aid of proteases that are naturally present in the yeast cells, the enzymatic degradation method of lysing cell bodies by adding an enzyme preparation derived from a microorganism or a plant, the hot water extraction method of lysing cell bodies by soaking cells in hot water for a fixed period of time, the acid/alkali decomposition method of lysing cell bodies by adding various types of acids or alkalis, the freezing and thawing method of disrupting cell bodies by conducting one or more times of freezing and thawing, the physical disruption method of disrupting cell bodies by a physical stimulation, or the like. The physical stimulation to be used in the physical disruption method can be exemplified by ultrasonic treatment, homogenization under high pressure, grinding by mixing with a solid material such as glass beads, or the like.

In addition, dry yeast cells having a high glutathione content can also be obtained by drying a culture product obtained by the method of culturing a yeast disclosed herein. The method of drying the culture product is not specifically limited, and any method which is usually conducted for preparing dry yeast cells can be adopted. The preparation method can be exemplified by the freeze dry method, the spray dry method, the drum dry method, or the like. Furthermore, it is also possible, by powdering the thus obtained dry yeast cells, to produce a dry yeast powder having a high glutathione content with good handleability.

In addition, it is also possible to obtain a fraction which contains glutathione from a culture product obtained by the method of culturing a yeast disclosed herein. The method of fractionating the glutathione-containing fraction from the culture product may be any usually conducted method. For example, it is possible, by fractionating an extract obtained by hot water extraction or another type of extraction by means of cell disruption with use of an affinity column that holds a highly affinitive substance for sulfur-containing compounds, to concentrate and purify it in the form of a fraction which contains a high concentration of glutathione.

Such a yeast of high glutathione content, dry yeast cells of the yeast, a yeast extract prepared from the yeast, and a powder of the yeast extract, obtained by the method of culturing a yeast disclosed herein may be in the form of a seasoning composition. The seasoning composition may be solely composed of the yeast extract, or the like, or may contain another component such as a stabilizer and a preservative, in addition to the yeast extract, or the like, disclosed herein.

The seasoning composition can be suitably used for various kinds of foods and drinks, similar to other seasoning compositions.

Furthermore, such a yeast of high glutathione content, dry yeast cells of the yeast, a yeast extract prepared from the yeast, and a powder of the yeast extract, obtained by the method of culturing a yeast disclosed herein, can also be directly mixed, as a starting material, in a food or drink. By so doing, a food or drink containing a high concentration of glutathione can be effectively produced. Such a food or drink can be any type of food or drink as long as it is usually possible to add a dry yeast, a yeast extract, or a seasoning composition containing any of them, to the food or drink. The type of food or drink can be exemplified by alcoholic drinks, soft drinks, fermented foods, seasonings, various kinds of soups, various kinds of bakeries, various kinds of confectioneries, and the like. Besides, the above-mentioned culture product and the like can also be processed in the form of a soft capsule, a hard capsule, a compressed tablet, or the like, to be taken as a supplement or the like.

Specifically speaking, a food or drink containing a high concentration of glutathione can be produced by adding the above-mentioned yeast of high glutathione content, dry yeast cells of the yeast, a yeast extract prepared from the yeast, a powder of the yeast extract, or the like, in the same manner as for adding other raw materials, during the production process of food or drink.

### [Examples]

Next is a more detailed description of the present invention with reference to Examples. Note that, in the Examples below, the term "total glutathione content per dry cell weight (% (w/w))" may be abbreviated as "GSH content rate (%)".

Moreover, among the yeasts used in the following Examples, *Saccharomyces cerevisiae* strain YNN27, *Saccharomyces cerevisiae* strain BY4742, *Saccharomyces cerevisiae* strain NCYC506, and *Schizosaccharomyces pombe* strain JCM1846 are wild-type strains.

The *Saccharomyces cerevisiae* strain AB9 <MATa/α gpi10/gpi10 ura3/URA3 leu2/LEU2> is a mutant strain which is capable of releasing a mannan protein into a medium, owing to a mutation in a gene encoding α,2-mannosyltransferase (for example, refer to the pamphlet of PCT International Publication Number WO/2006/025295). The *Saccharomyces cerevisiae* AB9 strain is deposited with International Patent Organism Depositary, National Institute of Advanced Industrial Science and Technology (1-1-1 Higashi, Tsukuba-shi, Ibaraki-ken) with the accession number of FERM BP-10390 (originally deposited on July 13, 2004 and transferred to the International Depository on August 3, 2005).

The *Saccharomyces cerevisiae* strain AB13 is a mutant strain of high glutathione content produced by the following manner. Firstly, the wild-type strain of *Saccharomyces cerevisiae* was treated with EMS, and mutant strains showing higher glutathione contents than that of the patent strain were selected from the resulting mutant strains. Next, the thus selected mutant strains were treated with EMS, and mutant strains showing higher glutathione contents than that of the patent strain were selected from the resulting mutant strains. *Saccharomyces cerevisiae* strain AB13, strain KK101, strain KK122, and strain KK124 were obtained through twice or more times of repetition of this process.

In addition, Tables 1 to 7 respectively show the composition of the semisynthetic medium, the YPD medium, the SD medium, and the molasses medium ((1) and (2)) used for the culture of yeasts in the following Examples, and the composition of trace elements and vitamin solutions added thereto.

**[Table 1]**

| Semisynthetic medium | Concentration (g/L) |
|---|---|
| KH₂PO₄ | 2.00 |
| MgSO₄·7H₂O | 0.50 |
| Meast Powder N | 0.59 |
| (NH₄)₂SO₄ | 5.00 |
| Trace Element (mL) | 10.00 |
| Vitamin Solution (mL) | 12.00 |
| Glucose | 30.00 |
| pH | 6.800 |
| Buffer | Na Citrate |

**[Table 2]**

| Trace Element | Concentration (g/L) |
|---|---|
| EDTA·2Na·2H₂O | 15.0 |
| ZnSO₄·7H₂O | 5.75 |
| MnCl₂·4H₂O | 0.32 |
| CuSO₄ ·5H₂O | 0.50 |
| CoCl₂ | 0.26 |
| Na₂MoO₄ ·2H₂O | 0.48 |
| CaCl₂ | 2.20 |
| FeSO₄·7H₂O | 2.80 |

**[Table 3]**

| Vitamin Solution | Concentration (g/L) |
|---|---|
| Biotin | 0.05 |
| Calcium pantothenate | 1.00 |
| Nicotinic acid | 1.00 |
| Myo-inositol | 25.0 |
| Thiamine hydrochloride | 1.00 |
| Pyridoxol hydrochloride | 1.00 |
| p-Aminobenzoic acid | 0.20 |

**[Table 4]**

| YPD medium | Reference Value Concentration (g/L) |
|---|---|
| Bacto yeast | 10.00 |
| Bacto peptone | 20.00 |
| Glucose | 20.00 |
| pH | 6.800 |

**[Table 5]**

| SD medium | Reference value Concentration (g/L) |
|---|---|
| Nitrogen base w/o AA | 6.70 |
| Glucose | 20.00 |
| pH | 6.800 |

**[Table 6]**

| Molasses medium (1) | Reference value Concentration (g/L) |
|---|---|
| KH₂PO₄ | 0.80 |
| MgSO₄ | 0.80 |
| Urea | 2.00 |
| (NH₄)₂SO₄ | 5.00 |
| Molasses (%: Sugar concentration) | 30.00 |
| pH | 6.800 |

**[Table 7]**

| Molasses medium (2) | Reference value Concentration (g/L) |
|---|---|
| KH₂PO₄ | 0.80 |
| MgSO₄ ·7H₂O | 0.28 |
| Mollasses | 180.00 |
| 85% Phosphoric acid | 1.68 |
| 10% Ammonia water | 8.20 |
| pH | 5.800 |

### <Example 1>

*Saccharomyces cerevisiae* strain KK101 was subjected to the yeast culture by a method of culturing a yeast with use of the semisynthetic medium, and then the GSH content rate (%) of the cultured yeast was measured.

Firstly, the strain KK101 having been preserved in glycerol at -80°C was smeared on a YPD agar medium and incubated at 30°C for 3 days. Then, a loop of the yeast that had grown on the YPD agar medium was inoculated in a 13 mL Assist Tube containing 3 mL of YPD medium, and subjected to shaking culture at 30°C with a shaking rate of 200 rpm for 1 to 3 days. The thus obtained culture solution was used as a preculture solution for the following main culture.

150 µL of the preculture solution was respectively inoculated in a 200 mL baffled Erlenmeyer flask containing 15 mL of a semisynthetic medium which had been added with citric acid so that the concentration would be 0 to 100 mM, and subjected to shaking culture at 30°C with a shaking rate of 200 rpm for 48 hours.

The total amount of glutathione contained in the yeast cells in the thus obtained culture product was measured by the method of Titze et. al. (Analytical Biochemistry, Vol. 27, p. 502, 1969). The measured value was divided by the dry cell weight. By so doing, the GSH content rate (total glutathione content per dry cell weight) was calculated. Specifically speaking, firstly, the obtained culture product was centrifuged at 4°C and 6000 rpm for 5 minutes. The recovered yeast cells were washed with purified water twice. To the yeast cells was added 2 mL of purified water, and the mixture was suspended. Then, 500 µL of this suspension was charged in an aluminum plate and dried at 105°C for 5 hours or longer, and furthermore, the plate was left still in a desiccator for 1 hour or longer. Thereafter, the dry weight was measured. The thus measured value was adopted as the dry cell weight. On the other hand, another 500 µL of the suspension was boiled in a hot water bath for 5 minutes. Immediately thereafter, the suspension was cooled down in an ice bath, and centrifuged at 4°C and 15000 rpm for 5 minutes. The recovered supernatant was used as a sample for the quantitation of glutathione. To 2.5 mL of 0.5M potassium phosphate buffer (pH7.0) having 1 mM EDTA mixed therein were added 5 µL of glutathione reductase and 500 µL of NADPH. The resultant solution was charged in a 4 mL disposal cell. Immediately before the measurement, 10 µL of the sample and 100 µL of DTNB were added to the cell, and the mixture was mixed well by inverting the cell. For 30 seconds after the initiation of the reaction, the increase of the absorption at 412 nm was measured by a spectrophotometer. The amount of glutathione was calculated from the obtained measurement value by using a calibration curve that had been previously prepared from measurement values given by experiments conducted in the presence of known concentrations of glutathione. The thus calculated value was adopted as the total amount of glutathione in the culture product. This total amount of glutathione was divided by the dry cell weight that had been measured separately. By so doing, the GSH content rate (%) was calculated.

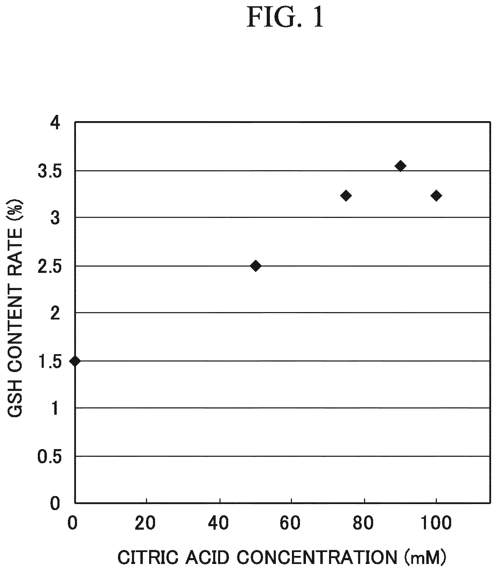

FIG. 1 is a graph showing the GSH content rate (%) with respect to the concentration of citric acid in the liquid medium. As a result, it was found that the addition of citric acid to the liquid medium led to an increase of the GSH content rate (%). The GSH content rate (%) was increased dependently on the citric acid concentration, and showed the maximum value when the citric acid concentration was 90 mM, with the condition of this Example.

### <Example 2>

*Saccharomyces cerevisiae* strain AB13 was subjected to the yeast culture by a method of culturing a yeast with use of the molasses medium (1), and then the GSH content rate (%) of the cultured yeast was measured.

150 µL of the preculture solution (1) that had been prepared in the same manner as that of Example 1 was respectively inoculated in a 200 mL baffled Erlenmeyer flask containing 15 mL of a molasses medium (1) which had been added with citric acid so that the concentration of the added citric acid would be 0 to 60 mM, and subjected to shaking culture at 30°C with a shaking rate of 200 rpm for 48 hours. Since a few mM of citric acid had been naturally included in the molasses medium from the beginning, the actual citric acid concentration in the molasses medium would have been slightly higher than the concentration of the added citric acid. The GSH content rate (%) of the cultured yeast was measured in the same manner as that of Example 1. Furthermore, the pH of the liquid medium at the completion of the culture was also measured.

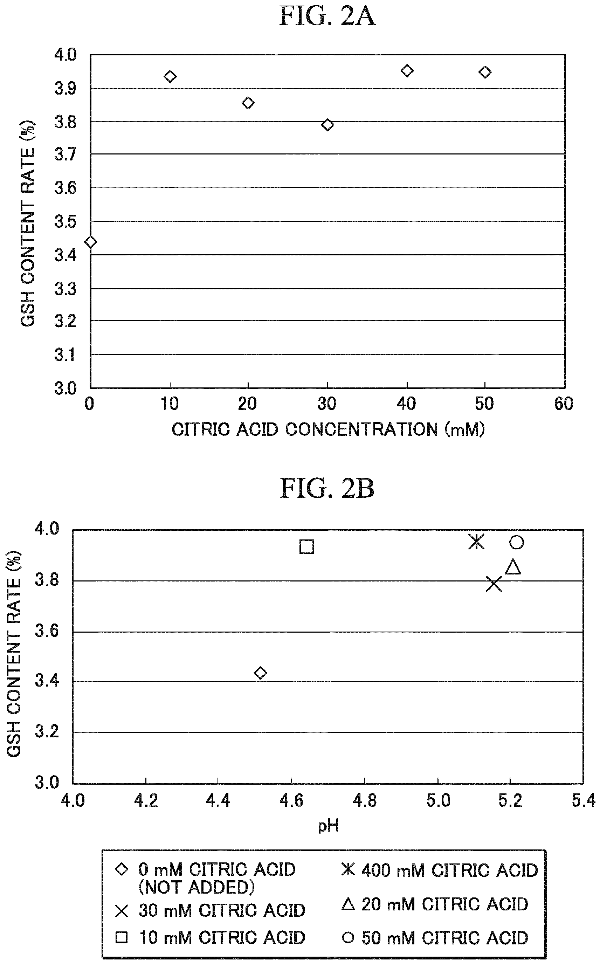

FIG. 2A and FIG. 2B are graphs showing the GSH content rate (%) of the respective culture products (FIG. 2A), and the pH of the liquid medium at the completion of the culture (final pH) (FIG. 2B), with respect to the concentration of citric acid added to the molasses medium (1) in Example 2. As a result, the addition of 10 mM or more of citric acid to the molasses medium (1) was able to increase the GSH content rate (%) of the yeast by 10% or higher than before the addition. Moreover, the pH of the liquid medium at the completion of the culture was increased by the addition of citric acid. However, when the amount of the added citric acid was equal to or greater than 20 mM, the pH became stable at around pH5.2.

### <Example 3>

*Saccharomyces cerevisiae* strain KK101 was cultured with use of semisynthetic media to which various types of acids had been added. The GSH content rates (%) of the cultured yeast of these cases were measured so as to investigate the influence of the respective types of acids in the liquid medium on the glutathione content of the yeast.

150 µL of the preculture solution that had been prepared in the same manner as that of Example 1 was respectively inoculated in a 200 mL baffled Erlenmeyer flask containing 15 mL of a semisynthetic medium which had been added with respective types of salts shown in Table 8 so that the concentration thereof would be that of the table, and subjected to shaking culture at 30°C with a shaking rate of 200 rpm for 48 hours.

The GSH content rate (%) of the cultured yeast was measured in the same manner as that of Example 1. Furthermore, the pH (final pH) and the OD₆₀₀ of the liquid medium at the completion of the culture were also measured. The measured results are shown in Tables 8 to 10.

**[Table 8]**

| GSH content rate (%) | | | | | | | |
|---|---|---|---|---|---|---|---|
| | Concentration (mM) | | | | | | |
| Types of acid | 50 | 75 | 90 | 112.5 | 135 | 150 | 200 |
| Citric acid | 2.8 | 3.1 | 3.4 | | | | |
| Isocitric acid | 2.8 | 2.9 | 2.9 | | | | |
| cis-Aconitic acid | 3.0 | 3.0 | 2.9 | | | | |
| Tricarballylic acid | 2.7 | 2.8 | 2.8 | | | | |
| Fumaric acid | | 2.8 | | 2.7 | 2.8 | | |
| Malic acid | | 2.8 | | 2.8 | 2.7 | | |
| Succinic acid | | 2.7 | | 2.7 | 2.8 | | |
| Phosphoric acid | 2.4 | 2.8 | 2.9 | | | 2.9 | 3.0 |

**[Table 9]**

| Final pH | | | | | | | |
|---|---|---|---|---|---|---|---|
| | Concentration (mM) | | | | | | |
| Types of acid | 50 | 75 | 90 | 112.5 | 135 | 150 | 200 |
| Citric acid | 4.9 | 5.4 | 5.5 | | | | |
| Isocitric acid | 5.0 | 5.5 | 5.6 | | | | |
| cis-Aconitic acid | 4.6 | 5.3 | 5.4 | | | | |
| Tricarballylic acid | 5.0 | 5.4 | 5.6 | | | | |
| Fumaric acid | | 4.3 | | 4.5 | 4.5 | | |
| Malic acid | | 4.6 | | 4.8 | 5.0 | | |
| Succinic acid | | 5.1 | | 5.5 | 5.5 | | |
| Phosphoric acid | 3.1 | 4.5 | 5.3 | | | 6.1 | 6.4 |

**[Table 10]**

| OD₆₀₀ | | | | | | | |
|---|---|---|---|---|---|---|---|
| | Concentration (mM) | | | | | | |
| Types of acid | 50 | 75 | 90 | 112.5 | 135 | 150 | 200 |
| Citric acid | 31.2 | 29.6 | 27.9 | | | | |
| Isocitric acid | 30.9 | 28.7 | 28.8 | | | | |
| cis-Aconitic acid | 30.4 | 30.0 | 30.4 | | | | |
| Tricarballylic acid | 30.2 | 33.1 | 29.0 | | | | |
| Fumaric acid | | 32.1 | | 31.8 | 30.9 | | |
| Malic acid | | 32.0 | | 30.2 | 30.5 | | |
| Succinic acid | | 29.8 | | 29.6 | 27.7 | | |
| Phosphoric acid | 30.5 | 26.5 | 27.5 | | | 28.2 | 24.7 |

As a result, it was found that: in the cases of acids other than phosphoric acid, the pH of the liquid medium was adjusted to 4.0 to 6.0 by the addition of equal to or greater than 50 mM of the acid; and that the dose of the acid within this range did not impose so much influence on the pH of the liquid medium. It was also found that: in the cases of phosphoric acid, the pH of the liquid medium was increased dependently on the dose; and that the dose had a great influence on the pH of the liquid medium.

In addition, in all cases of using any type of the liquid medium, the OD₆₀₀ value at the completion of the culture was more or less 30, showing that the type and the dose of acid added to the liquid medium had little influence on the viability of the yeast.

On the other hand, in all cases of acids other than citric acid, the GSH content rate was more or less 2.8%, meaning that there was no particular difference. With the liquid medium in which the phosphoric acid concentration was set to be 50 mM, the GSH content rate was 2.4%, which was a value lower than the other cases. It is suggested that this is due to the pH of the liquid medium being lower than 4. Conversely, the liquid medium containing citric acid showed a tendency to have a higher GSH content rate dependent on the citric acid content.

From these results, it is apparent that the glutathione-increasing effect achieved by culturing the yeast in a citric acid-containing medium is an effect peculiar to citric acid. The GSH content rate and the final pH of the liquid medium were almost equivalent between both cases where the citric acid concentration of the liquid medium was set to be 50 mM and where 50 mM or more of another type of acid was added. This suggests that, when using the semisynthetic medium, the majority of the citric acid that had been added so that the citric acid concentration would be 50 mM was utilized for the adjustment of the pH of the liquid medium.

### <Example 4>

*Saccharomyces cerevisiae* strain KK101 was subjected to the yeast culture by a method of culturing a yeast with use of a liquid medium to which additional citric acid has been added under a pH-controlled condition, and then the GSH content rate (%) of the cultured yeast was measured.

150 µL of the preculture solution that had been prepared in the same manner as that of Example 1 was respectively inoculated in a 200 mL baffled Erlenmeyer flask containing 15 mL of a semisynthetic medium which had been added with respective types of acids shown in Table 11 so that the succinic acid concentration and the citric acid concentration would be that of the table, and subjected to shaking culture at 30°C with a shaking rate of 200 rpm for 48 hours. The GSH content rate (%) of the cultured yeast was measured in the same manner as that of Example 1.

Furthermore, the pH of these liquid media at the completion of the culture (final pH) was also measured. The measured results are shown in Table 11.

As a result, it was found that the GSH content rate was dramatically increased by the addition of additional citric acid to the liquid medium, the pH of which had been adjusted to 5.5 to 6.0 by adding succinic acid, much better than the case where the citric acid was not added. Specifically speaking, in the case where the citric acid concentration was 0 mM (not added), the GSH content rate was 2.8%; while, in the case where the citric acid concentration was 50 mM, the GSH content rate was 3.7%, meaning 30% or more increase. On the other hand, in both cases where the citric acid concentration was respectively 75 mM and 90 mM, the GSH content rate was 4.3%, meaning a 50% or more increase higher than the case where the citric acid was not added.

In comparison with the results of Example 3, the GSH content rate was higher in this Example where succinic acid was additionally added, even under the same citric acid concentration in the liquid medium. This comparison also implies that a part of the citric acid added to the liquid medium was utilized for controlling the pH of the liquid medium in Example 3.

That is to say, it is apparent from these results that a higher glutathione-increasing effect was achieved by the addition of citric acid to the liquid medium whose pH had been controlled at 4.0 to 6.0, rather than the case where citric acid was added to the liquid medium whose pH had not been controlled at 4.0 to 6.0.

**[Table 11]**

| Flask No. | Succinic acid concentration (mM) | Citric acid concentration (mM) | GSH content rate (%) | Final pH |
|---|---|---|---|---|
| 1 | 135 | 0 | 2.8 | 5.6 |
| 2 | 135 | 50 | 3.7 | 5.7 |
| 3 | 135 | 75 | 4.3 | 5.9 |
| 4 | 135 | 90 | 4.3 | 5.9 |

### <Example 5>

*Saccharomyces cerevisiae* strain KK122 and strain KK124 were respectively subjected to the yeast culture by a method of culturing a yeast with use of a jar fermenter under pH-controlled culture conditions, and then the GSH content rates (%) of the cultured yeasts were measured.

Firstly, pre-preculture solutions of *Saccharomyces cerevisiae* strain KK122 and strain KK124 were respectively prepared in the same manner as that of Example 1. 100 µL of each pre-preculture culture solution was inoculated in a 200 mL baffled Erlenmeyer flask containing 10 mL of YPD medium, and subjected to shaking culture at 30°C with a shaking rate of 200 rpm for 1 day. The thus obtained culture solution was used as a preculture solution for the following main culture.

10 mL of each preculture solution was inoculated in a 2L jar fermenter containing 1L of a semisynthetic medium or a semisynthetic medium which had been added with sodium citrate (90 mM citric acid-containing medium) so that the citric acid concentration would be 90 mM, and subjected to aeration and shaking culture starting from pH 6.8 with a shaking rate of 300 rpm and an aeration rate of 1 V.V.M. at 30°C for 48 hours. During the culture, the pH was adjusted with IN sodium hydroxide solution so that the minimum pH value would be 6.0. Sampling was conducted and the OD₆₀₀ was measured every 12 hours from the initiation of the culture. Moreover, samples respectively taken 24, 36, and 48 hours after the initiation of the culture were also measured for the GSH content rate in the same manner as that of Example 1.

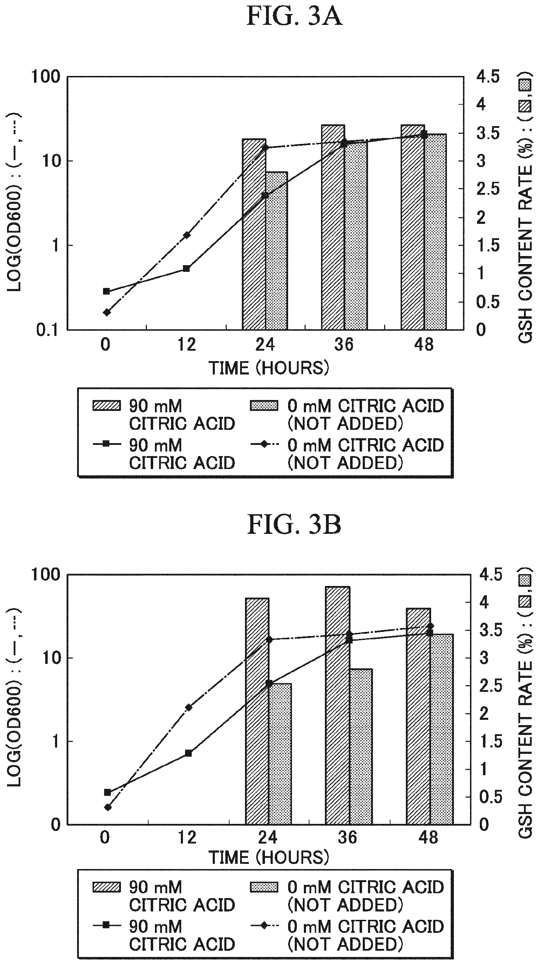

FIG. 3A and FIG. 3B show the measurement results of the GSH content rate and the OD₆₀₀ of *Saccharomyces cerevisiae* strain KK122 (FIG. 3A) and strain KK124 (FIG. 3B). In the graphs, the bar charts show the results of the GSH content rate (%), and the line charts show the results of the OD₆₀₀. Moreover, the term "90 mM citric acid" denotes the results obtained from the culture in the 90 mM citric acid-containing medium, and the term "0 mM citric acid (not added)" denotes the results obtained from the culture in the semisynthetic medium. As a result, the GSH content rates of *Saccharomyces cerevisiae* strain KK122 and strain KK124 were increased by the culture in the citric acid-containing liquid medium, similarly to the case of *Saccharomyces cerevisiae* strain KK101. In addition, although the inclinations of the OD₆₀₀ charts of both strains were slightly smaller when cultured in the 90 mM citric acid-containing medium than the cases when cultured in the semisynthetic medium, the OD₆₀₀ values at the completion of the culture were equivalent. From these results, it was found to be possible to culture these yeasts so that they could in the end act in a similar manner to the cases without the addition of citric acid, although the initial rise was shown to be slightly slower by the addition of citric acid to the liquid medium. This confirmed that the influence of the citric acid addition on the viability of the yeasts was small.

### <Example 6>

Various types of yeasts belonging to the genus *Saccharomyces* were subjected to the yeast culture by a method of culturing a yeast with use of the semisynthetic medium, and then the GSH content rates (%) of the cultured yeasts were measured. Based on the results of Example 3, the case where the citric acid concentration was set to be 50 mM was adopted as the reference (control), and the exertion of the glutathione-increasing effect was set to be notified when an increase of the GSH content rate in a dependent manner on the citric acid concentration was seen.

Firstly, preculture solutions of the strains shown in Table 12 were respectively prepared in the same manner as that of Example 1. Next, 150 µL of each preculture solution was respectively inoculated in a 200 mL baffled Erlenmeyer flask containing 15 mL of a semisynthetic medium that had been added with citric acid so that the citric acid concentration would be 50 to 150 mM, and subjected to shaking culture at 30°C with a shaking rate of 200 rpm for 48 hours. The GSH content rates (%) of the cultured yeasts were measured in the same manner as that of Example 1.

The measured results are shown in Table 12. As a result, not only mutant strains, namely, *Saccharomyces cerevisiae* strain KK101, strain KK122, strain KK124, and strain AB13, but also wild-type strains, namely, *Saccharomyces cerevisiae* strain YNN27, strain BY4742, and strain NCYC506, showed 10% or higher GSH content rates in the cases where the citric acid concentrations were between 75 and 150 mM rather than the case where the citric acid concentration was 50 mM. From these results, it is apparent that the glutathione-increasing effect of the present disclosure was achievable by applying the method of culturing a yeast disclosed herein or of the invention, not only in the cases of some specific types of mutant strains, but also in the cases of wild-type strains.

**[Table 12]**

| GSH content rate (%) | | | | |
|---|---|---|---|---|
| *Saccharomyces cerevisiae* | Citric acid concentration (mM) | | | |
| Strain name | 50 | 75 | 90 | 150 |
| Strain KK101 | 2.50 | 3.23 | 3.57 | |
| Strain KK122 | 2.86 | | 3.51 | 3.51 |
| Strain KK124 | 2.94 | | 3.27 | 3.52 |
| Strain AB13 | 2.94 | 3.27 | 3.52 | |
| Strain YNN27 | 0.36 | 0.45 | 0.51 | |
| Strain BY4742 | 0.42 | 0.66 | 0.65 | |
| Strain NCYC506 | 1.38 | 1.61 | 1.56 | |

### <Example 7>

Various types of yeasts belonging to the genus *Saccharomyces* were subjected to the yeast culture by a method of culturing a yeast with use of the YPD medium, and then the GSH content rates (%) of the cultured yeasts were measured. The exertion of the glutathione-increasing effect of the present disclosure or invention was set to be notified when an increase of the GSH content rate in a dependent manner on the citric acid concentration was seen.

Firstly, preculture solutions of the strains shown in Table 13 were respectively prepared in the same manner as that of Example 1. Next, 150 µL of each preculture solution was respectively inoculated in a 200 mL baffled Erlenmeyer flask containing 15 mL of YPD medium that had been appropriately added with citric acid so that the citric acid concentration would be 0 to 90 mM, and subjected to shaking culture at 30°C with a shaking rate of 200 rpm for 48 hours. The GSH content rates (%) of the cultured yeasts were measured in the same manner as that of Example 1.

The measured results are shown in Table 13. As a result, it was found that the glutathione-increasing effect was achievable also in the YPD medium, in both cases of the respective mutant strains and wild-type strains of *Saccharomyces cerevisiae.*

**[Table 13]**

| GSH content rate (%) | | | | | |
|---|---|---|---|---|---|
| *Saccharomyces cerevisiae* | Citric acid concentration (mM) | | | | |
| Strain name | 0 | 62.5 | 75 | 80 | 90 |
| Strain AB9 | 0.24 | | | 0.35 | 0.52 |
| Strain KK101 | 1.91 | 2.12 | 3.75 | | 3.68 |
| Strain KK124 | 2.16 | | | 3.69 | 4.49 |
| Strain AB13 | 1.91 | | | 2.07 | 2.31 |
| Strain YNN27 | 0.38 | | | 0.48 | 0.51 |

### <Example 8>

Another type of yeast differing from the genus *Saccharomyces* was also subjected to the yeast culture by a method of culturing a yeast, and then the GSH content rate (%) of the cultured yeast was measured. Specifically speaking, *Candida utilis* strain 1561 was cultured in a YPD medium containing citric acid, and then the GSH content rate thereof was measured.

Firstly, a preculture solution of *Candida utilis* strain 1561 was prepared in the same manner as that of Example 1. Next, 15 µL of the preculture solution was respectively inoculated in a 200 mL baffled Erlenmeyer flask containing 15 mL of YPD medium (inoculum concentration was 0.1%) that had been appropriately added with citric acid so that the citric acid concentration would be 0 to 62.5 mM, and subjected to shaking culture at 30°C with a shaking rate of 200 rpm for 40 hours. The GSH content rate (%) of the cultured yeast was measured in the same manner as that of Example 1.

The measured results are shown in Table 14. As a result, it was found that the glutathione-increasing effect was also achievable in the case of *Candida utilis* strain 1561, similarly to the cases of yeasts belonging to the genus *Saccharomyces.*

**[Table 14]**

| GSH content rate (%) of *Candida utilis* strain 1561 | | | |
|---|---|---|---|
| | Citric acid concentration (mM) | | |
| Culture time | 0 | 50 | 62.5 |
| 24 hours | 1.97 | 2.11 | |
| 40 hours | 1.71 | | 2.23 |

### <Example 9>

Another type of yeast belonging to the genus *Candida* was also subjected to the yeast culture by a method of culturing a yeast, and then the GSH content rate (%) of the cultured yeast was measured. Specifically speaking, *Candida utilis* strain 1560 was cultured in the molasses medium (2) containing citric acid, and then the GSH content rate thereof was measured.

Firstly, a preculture solution of *Candida utilis* strain 1560 was prepared in the same manner as that of Example 1. Next, 45 µL of the preculture solution was respectively inoculated in a 200 mL baffled Erlenmeyer flask containing 15 mL of a molasses medium (2) (inoculum concentration was 0.3%) that had been appropriately added with citric acid so that the citric acid concentration would be 0 to 62.5 mM, and subjected to shaking culture at 30°C with a shaking rate of 200 rpm for 24 hours. The GSH content rate (%) of the cultured yeast was measured in the same manner as that of Example 1.

The measured results are shown in Table 15. As a result, it was found that the glutathione-increasing effect was also achievable in the case of *Candida utilis* strain 1560, similarly to the cases of yeasts belonging to the genus *Saccharomyces.*

**[Table 15]**

| GSH content rate (%) of *Candida utilis* strain 1560 | | | |
|---|---|---|---|
| | Citric acid concentration (mM) | | |
| Culture time | 0 | 20 | 50 |
| 22 hours | 1.58 | | 1.69 |
| 24 hours | 1.77 | 1.81 | 1.82 |

### <Example 10>

Yeasts belonging to the genus *Saccharomyces* were subjected to the yeast culture by a method of culturing a yeast with use of the SD medium, and then the GSH content rate (%) of the cultured yeasts were measured. The exertion of the glutathione-increasing effect was set to be notified when an increase of the GSH content rate in a dependent manner on the citric acid concentration was seen.

Firstly, preculture solutions of the strains shown in Table 16 were respectively prepared in the same manner as that of Example 1. Next, 150 µL of each preculture solution was respectively inoculated in a 200 mL baffled Erlenmeyer flask containing 15 mL of a SD medium that had been appropriately added with citric acid so that the citric acid concentration would be 50 to 150 mM, and subjected to shaking culture at 30°C with a shaking rate of 200 rpm for 48 hours. The GSH content rates (%) of the cultured yeasts were measured in the same manner as that of Example 1.

The measured results are shown in Table 16. As a result, it was found that the glutathione-increasing effect was achievable for the respective strains of *Saccharomyces cerevisiae* even with the SD medium, in both cases of the mutant strains and the wild-type strains. From these results, it can be said that the glutathione-increasing effect is not specifically limited to the composition of the liquid medium to be used, but can be achieved by using any type of medium provided that a sufficient amount of citric acid is added.

**[Table 16]**

| GSH content rate (%) | | | |
|---|---|---|---|
| *Saccharomyces cerevisiae* | Citric acid concentration (mM) | | |
| Strain name | 50 | 75 | 90 |
| Strain KK101 | 2.33 | 2.37 | 2.45 |
| Strain YNN27 | 0.34 | 0.42 | 0.42 |
| Strain BY4742 | 0.33 | 0.51 | 0.67 |

### <Example 11>

Some yeasts were subjected to the yeast culture by a method of culturing a yeast by adjusting the citric acid concentration of the liquid medium to be equal to or greater than 20 mM, after the initiation of the culture, and then the GSH content rates (%) of the cultured yeasts were measured. Based on the results of Example 3, the case where the citric acid concentration at the initiation of the culture was set 50 mM was adopted as the reference (control). The exertion of the glutathione-increasing effect was set to be notified when the GSH content rate was higher than the reference case.

Preculture solutions of *Saccharomyces cerevisiae* strain KK101, strain KK122, strain KK124, and strain AB13 were respectively prepared in the same manner as that of Example 1.

Next, the respective strains were cultured in the following manner. Firstly, 150 µL of the preculture solution was respectively inoculated in five 200 mL baffled Erlenmeyer flasks respectively containing 15 mL of the semisynthetic medium. Two of these flasks were respectively added with citric acid so that the citric acid concentration would be 50 mM or 90 mM respectively (referred to as the "sample with 50 mM addition at initiation" and the "sample with 90 mM addition at initiation"). These five flasks were subjected to shaking culture at 30°C with a shaking rate of 200 rpm. At 3 hours after the initiation of the culture, one of the three flasks without the addition of citric acid was added with citric acid so that the citric acid concentration would be 90 mM and the culture was kept going (referred to as the "sample with 90 mM addition after 3 hours"). Moreover, at 6 hours after the initiation of the culture, one of the two flasks without the addition of citric acid was added with citric acid so that the citric acid concentration would be 90 mM and the culture was kept going (referred to as the "sample with 90 mM addition after 6 hours"). Lastly, at 9 hours after the initiation of the culture, the last flask without the addition of citric acid was added with citric acid so that the citric acid concentration would be 90 mM and the culture was kept going (referred to as the "sample with 90 mM addition after 9 hours"). At 48 hours after the initiation of the culture, the culture of all flasks was terminated. By so doing, five samples were obtained. This process of culture was conducted on every strain. By so doing, 20 samples in total were obtained.

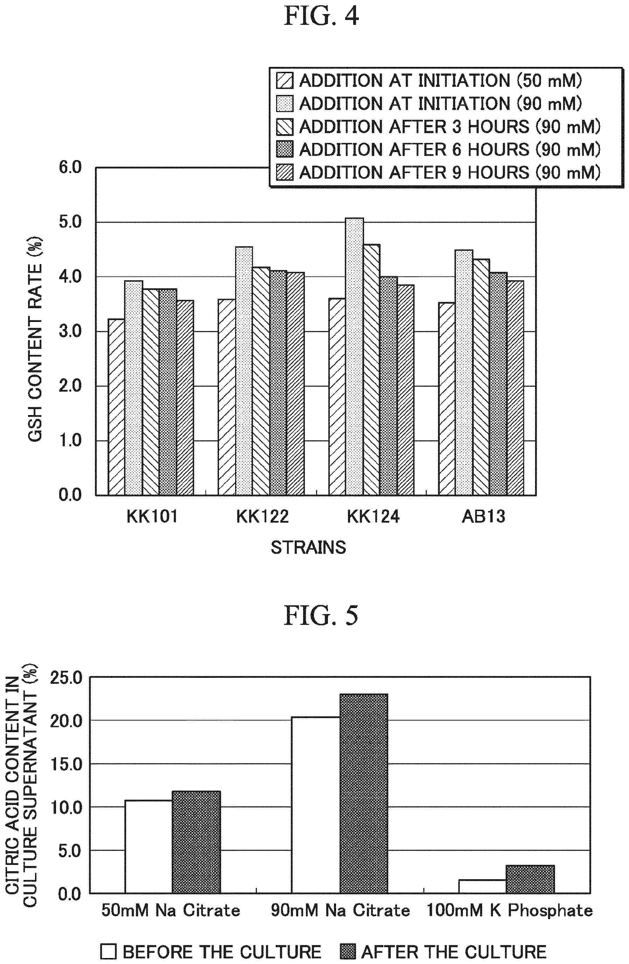

FIG. 4 shows the measurement results of the GSH content rates (%) of the yeasts in the respective samples.

The GSH content rate (%) was measured in the same manner as that of Example 1. As a result, with all strains, the GSH content rate of the sample with 90 mM citric acid addition, irrespective of the timing of the addition, was higher than that of the sample with 50 mM addition at initiation. In other words, it was confirmed from these results that the glutathione content of a yeast was able to be increased even though the citric acid concentration of the liquid medium had been adjusted after the initiation of the culture to a sufficient level so as to exert the glutathione-increasing effect. In addition, although slight differences between strains were seen, basically all strains showed a tendency such that the GSH content rate of the sample with 90 mM addition at initiation was the highest, and that the GSH content rate decreased as citric acid was added at a later timing. Thus, it was suggested that the adjustment of the citric acid concentration, if performed after the initiation of culture, is preferably conducted at least within 9 hours after the initiation of the culture, in other words, during the time when budding of the yeast is active.

### <Reference Example 1>

It was checked whether or not the citric acid having been added to a liquid medium was assimilated as a carbon source.

Firstly, the preculture solution of *Saccharomyces cerevisiae* strain KK101 was prepared in the same manner as that of Example 1.

150 µL of the preculture solution was inoculated in a 200 mL baffled Erlenmeyer flask respectively containing 15 mL of any one of: a semisynthetic medium which had been added with sodium citrate so that the citric acid concentration would be 50 mM (50 mM citric acid-containing medium); a semisynthetic medium which had been added with sodium citrate so that the citric acid concentration would be 90 mM (90 mM citric acid-containing medium); or a semisynthetic medium which had been added with potassium phosphate so that the phosphoric acid concentration would be 100 mM (100 mM phosphoric acid-containing medium), and subjected to shaking culture at 30°C with a shaking rate of 200 rpm for 48 hours. A part of the culture supernatant was previously collected after the inoculation of the yeast and before the culture. The citric acid content in the supernatant of the thus previously collected culture supernatant was measured, as well as that of the supernatant after the completion of the culture. The measurement of the citric acid content was conducted by the F-kit method using a commercially available kit (Product Name: F-kit Citric acid, manufactured by Roche).

FIG. 5 shows the measurement results of the citric acid content in the respective supernatants before and after the culture. In the graph, the term "50 mM Na Citrate" denotes the results obtained from the culture in the 50 mM citric acid-containing medium, the term "90 mM Na Citrate" denotes the results obtained from the culture in the 90 mM citric acid-containing medium", and the term "100 mM K Phosphate" denotes the results obtained from the culture in the 100 mM phosphoric acid-containing medium. As a result, the citric acid content in the supernatant after the culture was slightly higher than that before the culture, in all cases using any type of liquid medium. This suggests that the yeast might have generated citric acid during the process of culture. In other words, it is apparent from these results that the yeast did not assimilate the citric acid in the liquid medium and the citric acid having been added to the liquid medium was not used as a carbon source.

### INDUSTRIAL APPLICABILITY

The method of culturing a yeast of the present disclosure or invention is capable of readily and conveniently increasing the glutathione content of a yeast belonging to the genus *Saccharomyces* or such a yeast. Therefore, this culturing method is applicable particularly to the field of food production and so forth.

### [Accession Number]

FERM BP-10390

## Claims

1. A method of increasing a glutathione content of a yeast comprising:
culturing a yeast in a liquid medium whose citric acid concentration at an initiation of the culture is lower than 20 mM; and
adjusting the citric acid concentration of the liquid medium at 20 to 200 mM when a proliferative status of the yeast is in an induction phase or a logarithmic growth phase;
wherein the yeast belongs to *Saccharomyces cerevisiae.*

## Patentansprüche

1. Verfahren zur Erhöhung eines Glutathion-Gehalts einer Hefe, umfassend:
Züchten einer Hefe in einem flüssigen Medium, dessen kritische Zitronensäurekonzentration bei einer Initiierung der Kultur niedriger ist als 20 mM; und
Anpassen der kritischen Zitronensäurekonzentration des flüssigen Mediums auf 20 bis 200 mM, wenn ein Proliferationsstatus der Hefe sich in einer Induktionsphase oder einer logarithmischen Wachstumsphase befindet;
wobei die Hefen zu *Saccharomyces Cerevisiae* gehören.

## Revendications

1. Procédé d'augmentation d'une teneur en glutathione d'une levure comprenant :
la culture d'une levure dans un milieu liquide dont la concentration en acide citrique en début de culture est inférieure à 20 mM ; et
l'ajustement de la concentration en acide citrique du milieu liquide à 20 à 200 mM lorsqu'un statut de prolifération de la levure est en phase d'induction ou en phase de croissance logarithmique ;
dans lequel la levure appartient à *Saccharomyces cerevisiae.*
